# EUROPEAN PATENT APPLICATION

(11) **EP 3 904 317 A1**
(43) Date of publication of application: **03.11.2021**
(21) Application number: 19904958.6
(22) Date of filing: 11.12.2019
(51) Int. Cl.: C07B 53/00, C07C 269/06, C07C 271/22, C07K 1/02, C07K 1/10

(54) **METHOD FOR PRODUCING AMIDE**

(30) Priority: 25.12.2018 JP 2018241598
(71) Applicant: Tokyo Institute of Technology, Tokyo 152-8550 (JP); Yokogawa Electric Corporation, Tokyo 180-8750 (JP)
(72) Inventor: FUSE Shinichiro, Tokyo 152-8550 (JP); OTAKE Yuma, Tokyo 152-8550 (JP); NAKAMURA Hiroyuki, Tokyo 152-8550 (JP)
(74) Representative: Winter, Brandl - Partnerschaft mbB
(86) International application number: PCT/JP2019/048458
(87) International publication number: WO 2020/137545

(57) **Abstract**

A method for producing an amide includes: subjecting carboxylic acids to dehydration condensation or causing a reaction between a carboxylic acid and a haloformic acid ester; and subsequently causing a reaction with a first base and a reaction with an amine to obtain an amide, wherein the reaction with a first base and/or the reaction with an amine is performed by adding an acid thereto.

## Description

### [Technical Field]

The present invention relates to a method for producing an amide.

Priority is claimed on Japanese Patent Application No. 2018-241598, filed December 25, 2018, the content of which is incorporated herein by reference.

### [Background Art]

In peptide synthesis, an amino acid is sequentially extended by repeating activation of a carboxyl group of the amino acid to cause a reaction with an amino group of the amino acid and formation of an amide bond due to a coupling reaction. Several methods are known as methods of activating a carboxyl group. There is a method of synthesizing peptides while suppressing isomerization or generation of by-products using a condensing agent with a low activation level or a method of synthesizing peptides in a short period of time using an activator.

There is an acid chloride method or an acid anhydride method as methods of activating a carboxyl group using a highly active activator. In such an acid chloride method or acid anhydride method, the structure of an activator is simple compared to the activation method in which a condensing agent with a low activation level is used, and therefore, there are advantages such as a low unit price and less production of by-products derived from an activator.

The acid anhydride methods are divided into a symmetrical acid anhydride method and a mixed acid anhydride method.

For example, a method for synthesizing an amide using a symmetrical acid anhydride as an active species of a carboxylic acid is disclosed in Non-Patent Literature 1 and 2.

It can be said that the symmetrical acid anhydride method disclosed in Non-Patent Literature 1 and 2 is a method including a first step of generating a symmetrical acid anhydride through a condensation reaction between carboxylic acids and a second step of causing a coupling reaction between the symmetrical acid anhydride and an amine.

In addition, a method for synthesizing an amide using a mixed acid anhydride as an active species of a carboxylic acid is disclosed in Non-Patent Literature 3.

Non-Patent Literature 3 discloses that a carboxylic acid is mixed with isopropyl chloroformate with a first micromixer to synthesize the mixed acid anhydride in a short period of time, a solution containing the mixed acid anhydride, an amine, and a catalyst (base) being subsequently immediately mixed with each other with a second micromixer so as not to cause racemization of the synthesized mixed acid anhydride, to perform amidation.

It can be said that the mixed acid anhydride method disclosed in Non-Patent Literature 3 is a method including a first step of causing a reaction between a carboxylic acid and a chloroformic acid ester to obtain a mixed acid anhydride, a second step of adding a base to the mixed acid anhydride to obtain an acylpyridinium species, and a third step of causing a coupling reaction between the acylpyridinium species and an amine to obtain an amide.

### [Citation List]

### [Non-Patent Literatures]

[Non-Patent Literature 1]
   "Efficient Amide Bond Formation through a Rapid and Strong Activation of Carboxylic Acids in a Microflow Reactor", Fuse, S. Mifune, Y. Takahashi, T., Angew Chem. Int. Ed. 53, 851-855 (2014).
[Non-Patent Literature 2]
   "Total synthesis of feglymycin based on a linear/convergent hybrid approach using micro-flow amide bond formation", Fuse, S. Mifune, Y. Nakamura, H. Tanaka, H. Nat. Commun. 7, 13491 (2016).
[Non-Patent Literature 3]
   "An Efficient Synthesis of N-Methylated Peptides Using Micro-Flow Technology", Yuma Otake, Hiroyuki Nakamura, Shinichiro Fuse, March 16, 2017, The 97th Annual Meeting of The Chemical Society of Japan, 3F4-14

### [Summary of Invention]

### [Technical Problem]

However, in the symmetrical acid anhydride method, since the reactivity of a symmetrical acid anhydride with an amine is low, there is a problem in that the coupling reaction with an amine having a low nucleophilicity takes time or does not proceed.

On the other hand, in the mixed acid anhydride method, the coupling reaction can proceed even with an amine having a low nucleophilicity.

However, epimers which are undesired side reaction products may be sometimes produced in both the symmetrical acid anhydride method and the mixed acid anhydride method. This is particularly problematic in the coupling reaction with an amine having a low nucleophilicity. For example, in a usual coupling reaction represented by a natural amino acid, there is a sufficient difference between the reaction rate of racemization of an amino acid and the reaction rate at which a target product is produced, and therefore, production of epimers hardly occurs. However, in a case of a reaction with N-methylamino acid or the like having a low nucleophilicity, there is not a sufficient difference between the reaction rate of racemization and the reaction rate at which a target product is produced, and therefore, production of epimers may not sometimes be suppressed. In particular, in a case where phenylglycine, cysteine, or the like which easily causes racemization is used as a carboxylic acid and N-methylamino acid or the like having a low nucleophilicity is used as an amine, there is a problem in that epimer production of amides significantly occurs.

The present invention has been made to solve the above-described problems, and an object of the present invention is to provide a method for producing an amide which has a favorable reaction efficiency and is unlikely to cause a side reaction such as production of epimers in reactions in which a carboxyl group is activated to cause a reaction with an amino group and an amide bond is formed due to a coupling reaction.

### [Solution to Problem]

The present inventors have conducted extensive studies to solve the above-described problems, and as a result, they have found that the production of epimers is suppressed by adding an acid to a reaction system, thus leading to realization of the present invention.

That is, one embodiment of the present invention is as described below.
(1) A method for producing an amide, the method including:
   subjecting carboxylic acids to dehydration condensation or causing a reaction between a carboxylic acid and a haloformic acid ester; and
   subsequently causing a reaction with a first base and a reaction with an amine to obtain an amide,
   wherein the reaction with a first base and/or the reaction with an amine is performed by adding an acid thereto.
(2) The method for producing an amide, the method including:
   mixing a product obtained by reacting a mixture obtained by mixing a first carboxylic acid with a second carboxylic acid or a product obtained by reacting a mixture obtained by mixing a carboxylic acid and a haloformic acid ester, with a first base, an acid, and an amine.
(3) The method for producing an amide according to the above-described (1) or (2),
   wherein phosgene or a phosgene equivalent that decomposes in a reaction system and produces phosgene is caused to react, and the carboxylic acids are subjected to dehydration condensation.
(4) The method for producing an amide according to any one of the above-described (1) to (3),
   wherein the same kinds of the carboxylic acids are subjected to dehydration condensation.
(5) The method for producing an amide according to the above-described (1) or (2),
   wherein the haloformic acid ester is any one or more kinds selected from the group consisting of isopropyl chloroformate, isobutyl chloroformate, ethyl chloroformate, isopropyl bromoformate, isobutyl bromoformate, and ethyl bromoformate.
(6) The method for producing an amide according to the above-described (1), (2), or (5), the method further including:
   causing a reaction between the haloformic acid ester and a second base that activates the haloformic acid ester.
(7) The method for producing an amide according to the above-described (6),
   wherein the second base is any one or more kinds selected from the group consisting of tertiary amines, 4-methylmorpholine, pyridine, pyridine derivatives, imidazole, imidazole derivatives, and 1,4-diazabicyclo[2,2, 2]octane.
(8) The method for producing an amide according to any one of the above-described (1) to (7),
   the acid is any one or more kinds selected from the group consisting of a hydrogen halide, perchloric acid, trifluoroacetic acid, trifluoromethanesulfonic acid, fluorosulfonic acid, and paratoluenesulfonic acid.
(9) The method for producing an amide according to any one of the above-described (1) to (8),
   wherein the carboxylic acids are amino acids or amino acid derivatives.
(10) The method for producing an amide according to any one of the above-described (1) to (9),
   wherein the carboxylic acids are phenylglycine, cysteine, serine, aspartic acid, histidine, or derivatives thereof.
(11) The method for producing an amide according to any one of the above-described (1) to (10),
   wherein the first base is any one or more kinds selected from the group consisting of pyridine, pyridine derivatives, imidazole, imidazole derivatives, and 1,4-diazabicyclo[2, 2, 2]octane.
(12) The method for producing an amide according to according to any one of the above-described (1) to (11),
   wherein the first base is any one or more kinds selected from the group consisting of 4-morpholinopyridine, N,N-dimethyl-4-aminopyridine, 4-pyrrolidinopyridine, pyridine, 4-methoxypyridine, imidazole, N-methylimidazole, and 1,4-diazabicyclo[2,2, 2]octane.
(13) The method for producing an amide according to any one of the above-described (1) to (12),
   wherein the amine is an amino acid or an amino acid derivative.
(14) The method for producing an amide according to any one of the above-described (1) to (13),
   wherein a nucleophilicity of the amine is lower than that of 18 kinds of amino acids excluding valine and isoleucine from 20 kinds of amino acids which constitute proteins and are encoded by genetic information.
(15) The method for producing an amide according to the above-described (13) or (14),
   wherein the amine is valine, isoleucine, an N-alkylated amino acid, or derivatives thereof.
(16) The method for producing an amide according to any one of the above-described (1) to (15),
   wherein the reaction with the amine is performed in a continuous flow reactor.
(17) The method for producing an amide according to the above-described (16),
   wherein the reaction between the carboxylic acids or the reaction between the carboxylic acid and the haloformic acid ester, and the reaction with the first base are further performed in the continuous flow reactor.

### [Advantageous Effects of Invention]

According to the present invention, it is possible to provide a method for producing an amide which has a favorable reaction efficiency and is unlikely to cause a side reaction such as production of epimers.

### [Brief Description of Drawings]

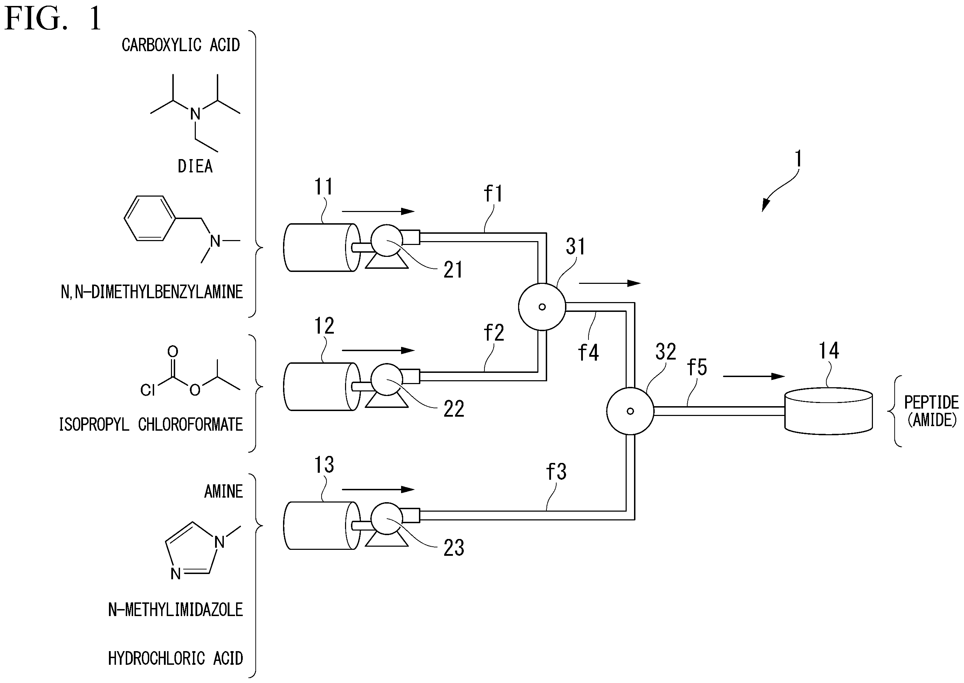

Fig. 1 is a schematic diagram illustrating a schematic configuration of a continuous flow reactor 1.

### [Description of Embodiments]

Hereinafter, an embodiment of a method for producing an amide of the present invention will be described.

### «Method for Producing Amide»

The method for producing an amide of the embodiment is the following method 1) or 2).
1) A method for producing an amide, the method including subjecting carboxylic acids to dehydration condensation and subsequently causing a reaction with a first base and a reaction with an amine to obtain an amide, in which the reaction with a first base and/or the reaction with an amine is performed by adding an acid thereto.
2) A method for producing an amide, the method including causing a reaction between a carboxylic acid and a haloformic acid ester and subsequently causing a reaction with a first base and a reaction with an amine to obtain an amide, in which the reaction with a first base and/or the reaction with an amine is performed by adding an acid thereto.

The method for producing an amide of the embodiment according to the above-described 1) may be a method for producing an amide, the method including mixing a product obtained by reacting a mixture obtained by mixing a first carboxylic acid with a second carboxylic acid, with a first base, an acid, and an amine. The method for producing an amide of the embodiment according to the above-described 1) may be a method including mixing a product obtained by reacting a mixture obtained by mixing a first carboxylic acid, a second carboxylic acid, and phosgene or a phosgene equivalent that decomposes in a reaction system and produces phosgene, with a first base, an acid, and an amine. Here, the product obtained by reacting a mixture obtained by mixing a first carboxylic acid and a second carboxylic acid may include an acid anhydride obtained by subjecting a first carboxylic acid and a second carboxylic acid to dehydration condensation.

The method for producing an amide of the embodiment according to the above-described 2) may be a method for producing an amide, the method including mixing a product obtained by reacting a mixture obtained by mixing a carboxylic acid and a haloformic acid ester, with a first base, an acid, and an amine. The method for producing an amide of the embodiment according to the above-described 2) may be a method for producing an amide, the method including mixing a product obtained by reacting a mixture obtained by mixing a carboxylic acid, a second base that activates a haloformic acid ester, and a haloformic acid ester, with a first base, an acid, and an amine. Here, the product obtained by reacting a mixture obtained by mixing a carboxylic acid and a haloformic acid ester or the product obtained by reacting a mixture obtained by mixing a carboxylic acid, a second base that activates a haloformic acid ester, and a haloformic acid ester may include a mixed acid anhydride.

The first base may be one that produces a cationically active species or may be a base (but excluding the amine).

The mixing referred to herein is an operation of adding substances such as raw materials to a reaction system, and when these are mixed with each other in a reaction system, the mixture may be converted to a substance different from those before the raw materials or the like are added thereto.

Examples of preparing an acid anhydride include a case of obtaining an acid anhydride through the method 1) and a case of obtaining an acid anhydride through the method 2) as described above. In both the methods 1) and 2) for producing an amide, it is common that an "acid anhydride" is obtained from a carboxylic acid to cause a reaction with a first base. That is, the methods 1) and 2) for producing an amide may include the following Steps 1 to 3. The reactions of the methods for producing an amide according to the present invention are not limited to the reactions exemplified in the following steps.
Step 1: A step of obtaining an acid anhydride from a carboxylic acid.
Step 2: A step of causing a reaction between the acid anhydride obtained in Step 1 and a first base to obtain a cationically active species.
Step 3: A step of producing an amide by causing a reaction between the cationically active species obtained in Step 2 and an amine.

However, in the above-described methods 1) and 2) for producing an amide, an acid may be added in the following reactions.

The reaction of Step 2 is performed through addition of an acid or the reaction of Step 3 is performed through addition of an acid, or the reactions of Steps 2 and 3 are performed through addition of an acid.

Although the addition of an acid may be previously performed in a stage of Step 1, an acid is preferably added after Step 1 and more preferably added together with a first base from the viewpoint of a reaction efficiency.

If an acid is added after Step 1, it is thought that the reaction of Step 2 or the reactions of Steps 2 and 3 occur in the presence of the acid. The presence of an acid means, for example, being in a solvent to which an acid is added.

In the method shown in the example to be described below, an acid is added together with a first base after Step 1 to produce an amide.

That is, regarding the addition of an acid, the following method 1') or 2') for producing an amide is preferable.
1') A method for producing an amide, the method including subjecting carboxylic acids to dehydration condensation and subsequently causing a reaction with a first base and a reaction with an amine through addition of an acid thereto to obtain an amide, in which the reaction with a first base and/or the reaction with an amine is performed by adding an acid thereto.
2') A method for producing an amide, the method including causing a reaction between a carboxylic acid and a haloformic acid ester and subsequently causing a reaction with a first base and a reaction with an amine through addition of an acid thereto to obtain an amide, in which the reaction with a first base and/or the reaction with an amine is performed by adding an acid thereto.

In the following embodiment, the case of adding an acid together with a first base will be mainly described.

### When adding an acid together with a first base, a mixture of a first base and an acid may be added.

Hereinafter, a case of obtaining an acid anhydride by subjecting carboxylic acids to dehydration condensation through the method 1') will be described as <First Embodiment>. In the present embodiment, the above-described Steps 1, 2, and 3 respectively correspond to Steps 1-1, 2-1, and 3-1.

In addition, a case of obtaining a mixed acid anhydride by causing a reaction between a carboxylic acid and a haloformic acid ester in the method 2') will be described as <Second Embodiment>. In the present embodiment, the above-described Steps 1, 2, and 3 respectively correspond to Steps 1-2, 2-2, and 3-2.

Hereinafter, in some cases, both Steps 1-1 and 1-2 may be simply abbreviated as Step 1, both Steps 2-1 and 2-2 may be simply abbreviated as Step 2, and both Steps 3-1 and 3-2 may be simply abbreviated as Step 3.

### <First Embodiment>

The production method 1') may include the following Steps 1-1 to 3-1.
Step 1-1: A step of subjecting carboxylic acids to dehydration condensation to obtain an acid anhydride.
Step 2-1: A step of causing a reaction between the acid anhydride obtained in Step 1-1 and a first base through addition of an acid to obtain a cationically active species.
Step 3-1: A step of producing an amide by causing a reaction between the cationically active species obtained in Step 2-1 and an amine.

Hereinafter, each of the above-described steps will be described. The reactions of the methods for producing an amide according to the present invention are not limited to the reactions exemplified in the following steps.

### <Step 1-1>

Step 1-1 is a step of subjecting carboxylic acids to dehydration condensation to obtain an acid anhydride.

Regarding the carboxylic acids, phosgene or a phosgene equivalent that decomposes in a reaction system and produces phosgene is caused to react, and the carboxylic acids are subjected to dehydration condensation.

A carboxylic acid may have a carboxyl group at a terminal of a molecule and can be represented by the following General Formula (1). 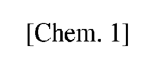 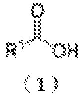 (In the formula, R¹ is a hydrogen atom or a monovalent organic group.)

A carboxylic acid may be deprotonated to form a carboxylate ion and can be represented by the following General Formula (1i). 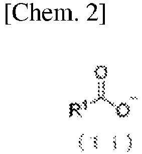 (In the formula, R¹ is a hydrogen atom or a monovalent organic group.)

The deprotonation of a carboxylic acid can be achieved by placing a carboxylic acid in the presence of a base, such as N,N-diisopropylethylamine (DIEA), having a low nucleophilicity in a reaction system.

The presence of a base means, for example, being in a solvent to which a base is added. The type of base is not particularly limited as long as the base can deprotonate a carboxylic acid in a reaction system.

In Step 1-1 of the method for producing an amide according to the embodiment, a carboxylic acid represented by the following General Formula (1) and a carboxylic acid represented by the following General Formula (1)' are subjected to dehydration condensation to obtain an acid anhydride represented by the following General Formula (2). The anhydride can be obtained by, for example, causing phosgene or a phosgene equivalent that decomposes in a reaction system and produces phosgene to react with a carboxylic acid. 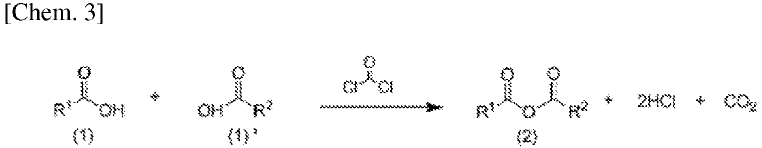 (In the formula, R¹ and R² each independently represent a hydrogen atom or a monovalent organic group.)

A phosgene equivalent decomposes in a reaction system to produce phosgene and can be used substantially as an equivalent of phosgene in a synthetic reaction. Examples of phosgene equivalents include diphosgene and triphosgene.

Regarding the dehydration condensation, different kinds of carboxylic acids may be subjected to dehydration condensation or the same kinds of carboxylic acids may be subjected to dehydration condensation. That is, R¹ and R² in Formulae (1) and (1)' may be the same as or different from each other.

In a case where R¹ and R² are the same as each other, an acid anhydride represented by General Formula (2) is a symmetrical acid anhydride. In the case where R¹ and R² are the same as each other, a counter anion of a cationically active species produced in Step 2-1 to be described below is the same as a carboxylate ion before activation. In some cases, a counter anion may self-react with a cationically active species. However, if the counter anion is the same as a carboxylate ion before activation, a product is the same as a symmetrical acid anhydride before activation into a cationically active species even if the counter anion self-reacts with the cationically active species.

Accordingly, in the case where R¹ and R² are the same as each other, there is an advantage that the types of amides obtained in a reaction system are homogeneous so that it is easy to systematically obtain desired types of reaction products.

The carboxylic acids are preferably amino acids or amino acid derivatives. The carboxylic acids here include carboxylic acids which are precursors of acid anhydrides. The amino acids are preferably α-amino acids. In addition, in general, since amino acids constituting peptides or proteins in a living body are L-types, the amino acids may be L-types. The α-amino acids may be compounds represented by the following General Formula (1-1). 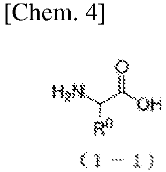 (In the formula, R⁰ represents a side chain of an amino acid.)

The amino acids may be 20 kinds of amino acids which constitute peptides or proteins and are encoded by genetic information in a living body. Examples of these amino acids include alanine, arginine, asparagine, aspartic acid, cysteine, glutamine, glutamic acid, glycine, histidine, isoleucine, leucine, lysine, methionine, phenylalanine, proline, serine, threonine, tryptophan, tyrosine, and valine. In addition, the amino acids may be types of amino acids, such as cystine, which are not encoded by genetic information.

For example, R⁰ in Formula (1-1) is "-CH₃" in a case where the amino acid is alanine, "-H" in a case where the amino acid is glycine, "-CH(CH₃)₂" in a case where the amino acid is valine, and "-CH(CH₃)CH₂CH₃" in a case where the amino acid is isoleucine. The same applies to other amino acids.

In a case where Formulae (1) and (1)' are amino acids, -R¹ and -R² may be - CH(R⁰)NH₂, respectively.

The amino acids may not be α-amino acids. For example, these may be β-amino acids such as β-alanine.

The carboxylic acids may be amino acid derivatives. Amino acid derivatives may be compounds having substantially the same properties as amino acids, natural-type compounds present naturally, or compounds having undergone alteration such as modification, addition, and substitution of functional groups unlike a natural type.

Examples of the case of the compounds having substantially the same properties as amino acids include a case of obtaining a compound by being incorporated into an enzyme having an amino acid as a substrate or a case of obtaining a compound by binding to a molecule that binds to an amino acid.

Examples of amino acid derivatives include ones in which one or more hydrogen atoms or groups of amino acids are substituted with other groups (substituents). Examples of amino acid derivatives include protected amino acids in which functional groups are protected by protective groups. Protective groups have an effect of inactivating reactive functional groups. It is also possible to deprotect protective groups and return the protected functional groups to a state before being protected. Here, the protection of functional groups means that atoms constituting the functional groups are substituted with protective groups. Examples of moieties protected by protective groups include one or more kinds of moieties selected from the group consisting of an amino group, a carboxyl group, and a side chain. Functional groups contained in side chains may be protected by protective groups at one site or two or more sites. In Step 1, amino groups and/or functional groups of side chains are preferably protected so as to prevent reactions of reactive functional groups other than carboxyl groups.

The types of protective group are not particularly limited and can be appropriately selected depending on the types of functional groups to be protected. Examples of protective groups of amino groups include carbamate protective groups, sulfonamide protective groups, acyl protective groups, or alkyl protective groups, but the present invention is not particularly limited thereto.

Examples of carbamate protective groups include a 2-benzyloxycarbonyl group (sometimes abbreviated as -Z or -Cbz), a tert-butoxycarbonyl group (sometimes abbreviated as -Boc), an allyloxycarbonyl group (sometimes abbreviated as -Alloc), a 2,2,2-trichloroethoxycarbonyl group (sometimes abbreviated as -Troc), a 2-(trimethylsilyl)ethoxycarbonyl group (sometimes abbreviated as -Teoc), a 9-fluorenylmethyloxycarbonyl group (sometimes abbreviated as -Fmoc), a p-nitrobenzyloxycarbonyl group (sometimes abbreviated as -Z(NO₂)), and a p-biphenylisopropyloxycarbonyl group (sometimes abbreviated as -Bpoc).

Examples of sulfonamide protective groups include a p-toluenesulfonyl group (sometimes abbreviated as -Ts or -Tos), a 2-nitrobenzenesulfonyl group (sometimes abbreviated as -Ns), 2,2,4,6,7-pentamethyldihydrobenzofuran-5-sulfonyl (sometimes abbreviated as -Pbf), 2,2,5,7,8-pentamethylchroman-6-sulfonyl (sometimes abbreviated as -Pmc), and 1,2-dimethylindole-3-sulfonyl (sometimes abbreviated as -MIS).

According to the method for producing an amide of the embodiment, epimer production of amides can be suppressed. Therefore, the method for producing an amide of the embodiment is suitable for a reaction with a carboxylic acid which is a raw material that easily causes epimer production of amides. Specific examples of carboxylic acids that easily cause epimer production of amides include phenylglycine, cysteine, serine, aspartic acid, histidine, or derivatives thereof.

### <Step 2-1>

Step 2-1 is a step of causing a reaction between the acid anhydride obtained in Step 1-1 and a first base through addition of an acid to obtain a cationically active species.

In Step 2-1 of the method for producing an amide according to the embodiment, an acid anhydride represented by the following General Formula (2) and a first base represented by B are caused to react with each other through addition of an acid represented by A to obtain a cationically active species represented by the following General Formula (4). In the reaction, a compound represented by the following General Formula (5) is produced as a counter anion of a cationically active species. 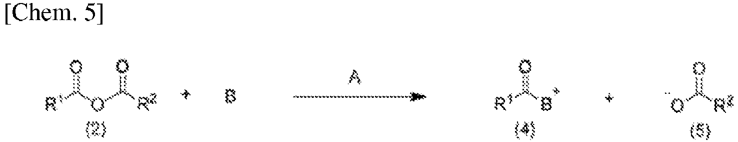 (In the formula, R¹ and R² each independently represent a hydrogen atom or a monovalent organic group.)

An acid to be added in Step 2-1 may be either an inorganic acid or an organic acid. Examples thereof include hydrochloric acid, hypochlorous acid, chlorous acid, chloric acid, perchloric acid, peroxo-monoperchloric acid, hydrobromic acid, hypobromous acid, bromic acid, hydrofluoric acid, nitric acid, sulfuric acid, persulfuric acid, phosphoric acid, perphosphoric acid, hexafluorophosphoric acid, potassium dihydrogen phosphate, sodium dihydrogen phosphate, hexafluoroantimonic acid, iodic acid, hypoiodous acid, periodic acid, boric acid, tetrafluoroboric acid, carbonic acid, percarbonic acid, permanganic acid, chromic acid, thiocyanic acid, acetic acid, peracetic acid, trifluoroacetic acid, chloroacetic acid, dichloroacetic acid, trichloroacetic acid, benzoic acid, perbenzoic acid, metachlorobenzoic acid, methanesulfonic acid, ethanesulfonic acid, benzenesulfonic acid, p-toluenesulfonic acid, trifluoromethanesulfonic acid, citric acid, formic acid, gluconic acid, lactic acid, oxalic acid, and tartaric acid, or salts obtained by reacting with these, but the present invention is not limited thereto.

An acid to be added here may be a carboxylic acid. Although a carboxylic acid is used in advance in Step 1-1, the carboxylic acid in Step 1-1 has become an acid anhydride and has been converted into an active species. Even if a carboxylic acid is added in Step 2-1, the added carboxylic acid is unlikely to become an active species, and the carboxylic acid added in Step 2-1 is unlikely to be incorporated as a constitutional unit of an amide. Therefore, the acid to be added here may be a carboxylic acid. However, the acid is preferably an acid excluding a carboxylic acid.

From the viewpoint of yield, the acid is preferably any one or more kinds selected from the group consisting of a hydrogen halide, perchloric acid, trifluoroacetic acid, trifluoromethanesulfonic acid, fluorosulfonic acid, and paratoluenesulfonic acid and more preferably a hydrogen halide. Among these, hydrochloric acid is more preferable.

By adding an acid, epimer production of amides can be suppressed, the coupling reaction rate between a carboxylic acid and an amine increases, and an amide can be produced with significantly high efficiency.

The first base in Step 2-1 reacts with the acid anhydride to produce a cationically active species, and is preferably one having a high nucleophilicity and more preferably any one or more kinds selected from the group consisting of pyridine, pyridine derivatives, imidazole, imidazole derivatives, and 1,4-diazabicyclo[2, 2, 2]octane.

Regarding pyridine derivatives, one or more hydrogen atoms of pyridine may be substituted with other groups. The present invention is not particularly limited as long as pyridine derivatives have properties of a base, but pyridine and pyridine derivatives are preferably compounds represented by the following General Formula (3-1). 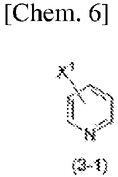 (In the formula, X¹ represents a hydrogen atom or any group selected from the group represented by the following Formulae (a) to (c).) 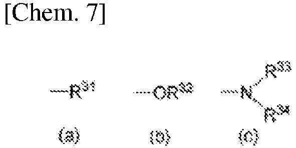 (In the formula, R³¹, R³², R³³, and R³⁴ each independently represent an alkyl group. R³³ and R³⁴ may bind to each other to form a ring, and one methylene group which has not directly bound to R³³ or R³⁴ in the alkyl group may be substituted with an oxygen atom.)

The alkyl groups of R³¹, R³², R³³, and R³⁴ may be either linear, branched, or cyclic. In the case of the cyclic shape, the alkyl group may be either monocyclic or polycyclic. The alkyl group may have 1 to 20 carbon atoms, 1 to 15 carbon atoms, or 1 to 10 carbon atoms.

Examples of the linear or branched alkyl group include a methyl group, an ethyl group, an n-propyl group, an isopropyl group, an n-butyl group, an isobutyl group, an sec-butyl group, a tert-butyl group, an n-pentyl group, an isopentyl group, a neopentyl group, a tert-pentyl group, a 1-methylbutyl group, an n-hexyl group, a 2-methylpentyl group, a 3-methylpentyl group, a 2,2-dimethylbutyl group, a 2,3-dimethylbutyl group, an n-heptyl group, a 2-methylhexyl group, a 3-methylhexyl group, a 2,2-dimethylpentyl group, a 2,3-dimethylpentyl group, a 2,4-dimethylpentyl group, a 3,3-dimethylpentyl group, a 3-ethylpentyl group, a 2,2,3-trimethylbutyl group, an n-octyl group, an isooctyl group, a nonyl group, a decyl group, a undecyl group, a dodecyl group, a tridecyl group, a tetradecyl group, a pentadecyl group, a hexadecyl group, a heptadecyl group, an octadecyl group, a nonadecyl group, and an icosyl group.

The compound represented by General Formula (3-1) is preferably a compound represented by the following General Formula (3-1-1). In a case where X¹ is any group selected from the group represented by Formulae (a) to (c) other than a hydrogen atom, X¹ effectively acts as an electron-donating group due to binding at the relevant position, and therefore, there is a tendency for N atoms of pyridine rings to have a more favorable nucleophilicity. 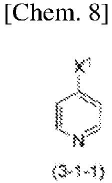 (In Formula (3-1-1), X¹ has the same meaning as X¹ in the above-described Formula (3-1).

The compounds represented by General Formula (3-1) include 4-morpholinopyridine represented by Formula (3-1-2) in a case where X¹ is a group represented by Formula (c), R³³ and R³⁴ bind to each other to form a ring, and one methylene group which has not directly bound to R³³ or R³⁴ in the alkyl group is substituted with an oxygen atom.

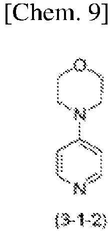

Preferred examples of the pyridine and pyridine derivatives include pyridine and the above-described 4-morpholinopyridine, N,N-dimethyl-4-aminopyridine, 4-pyrrolidinopyridine, and 4-methoxypyridine. Among these, 4-morpholinopyridine and N,N-dimethyl-4-aminopyridine are particularly preferable from the viewpoints that, due to use of these, the synthesis yield of amides per unit time is high and production of side reaction products can be significantly reduced.

A cationically active species in a case where pyridine and pyridine derivatives exemplified above are used is an acylpyridinium cation (acylpyridinium species). An acylpyridinium species has characteristics of having high electrophilicity. For this reason, even in a reaction with an amine having a low nucleophilicity, the reaction can proceed at a very high speed and the production of side reaction products can be significantly reduced.

Regarding imidazole derivatives, one or more hydrogen atoms of imidazole may be substituted with other groups. The present invention is not particularly limited as long as imidazole derivatives have properties of a base, but imidazole and imidazole derivatives are preferably compounds represented by the following General Formula (3-2). 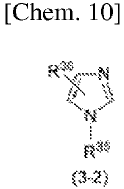 (In the formula, R³⁵ and R³⁶ each independently represent a hydrogen atom or an alkyl group.)

Those exemplified in the alkyl groups of R³¹, R³² , R³³ , and R³⁴ can be exemplified as alkyl groups of R³⁵ and R³⁶.

Preferred examples of the imidazole and imidazole derivatives include imidazole and N-methylimidazole.

Compounds represented by General Formula (3-2) include N-methylimidazole represented by the following Formula (3-2-1) in a case where R³⁶ is a hydrogen atom and R³⁵ is a methyl group.

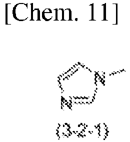

In addition, 1,4-diazabicyclo[2,2, 2]octane (DABCO) can be exemplified as a preferable one in addition to pyridine, pyridine derivatives, imidazole, and imidazole derivatives.

### <Step 3-1 >

Step 3-1 is a step of producing an amide by causing a reaction between the cationically active species obtained in Step 2-1 and an amine.

In Step 3-1 of the method for producing an amide according to the embodiment, a cationically active species represented by the following General Formula (4) and an amine represented by the following General Formula (6) are caused to react to obtain an amide represented by the following General Formula (7). Although not shown in the following formula, the acid added in the above-described Step 2-1 may be present in the reaction system. 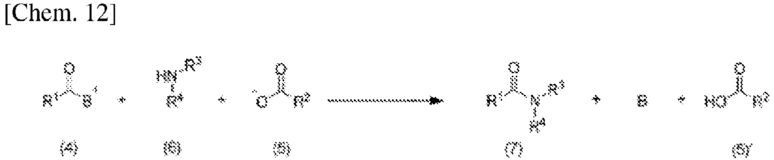 (In the formula, R¹, R², R³, and R⁴ each independently represent a hydrogen atom or a monovalent organic group.)

The amine is preferably an amino acid or an amino acid derivative.

Examples of amino acids and amino acid derivatives include those exemplified in the carboxylic acids.

In a case where Formula (6) is an amino acid, -R³ and -R⁴ may be, for example, -H and -CH(R⁰)COOH, respectively.

Examples of amino acid derivatives include protected amino acids in which functional groups are protected by protective groups. Examples of moieties protected by protective groups include one or more kinds of moieties selected from the group consisting of an amino group, a carboxyl group, and a side chain. Functional groups contained in side chains may be protected by protective groups at one site or two or more sites. In Step 3-1, carboxyl groups and/or functional groups of side chains are preferably protected so as to prevent reactions of reactive functional groups other than amino groups.

The types of protective group are not particularly limited and can be appropriately selected depending on the types of functional groups to be protected. In some cases, carboxyl groups may be protected simply by being neutralized in the form of salts. However, carboxyl groups are usually protected in the form of esters. Examples of esters include benzyl esters (sometimes abbreviated as Bn or BZl) in addition to alkyl esters such as methyl esters or ethyl esters, and the present invention is not limited thereto.

In the method for producing an amide of the embodiment, the cationically active species and an amine are caused to react in Step 3-1. Here, the method for producing an amide of the embodiment has an advantage that, due to high electrophilicity of the cationically active species, the reaction rate does not depend on a nucleophilicity of an amine.

Accordingly, the method for producing an amide of the embodiment is suitable for a reaction with an amine having a low nucleophilicity. An amine having a low nucleophilicity may be specifically an amine having a lower nucleophilicity than 18 kinds of amino acids excluding valine and isoleucine from 20 kinds of amino acids which constitute proteins and are encoded by genetic information. More specific examples thereof include valine, isoleucine, an N-alkylated amino acid, or derivatives thereof. In an N-alkylated amino acid, one or two hydrogen atoms of amino groups binding to α-carbons may be substituted with alkyl groups. N-methylamino acid in which one or two hydrogen atoms are substituted with methyl groups is preferable. It is difficult to use such amines having a low nucleophilicity for synthesis through an acid anhydride method in the related art. However, according to the method for producing an amide of the embodiment, amines having a low nucleophilicity which has been difficult to be used for synthesis through an acid anhydride method in the related art can be used, and the method for producing an amide of the embodiment is innovative in this respect.

The nucleophilicity of amines here can be obtained, for example, from the degree of efficiency of a reaction which is caused between an amine for which a nucleophilicity is desired and an acid anhydride produced in Example 1 through an acid anhydride method performed under the conditions shown in Example 1.

### <Second Embodiment>

The production method 2') may include the following Steps 1-2 to 3-2.
Step 1-2: A step of causing a reaction between a carboxylic acid and a haloformic acid ester to obtain a mixed acid anhydride.
Step 2-2: A step of causing a reaction between the mixed acid anhydride obtained in Step 1-2 and a first base through addition of an acid to obtain a cationically active species.
Step 3-2: A step of producing an amide by causing a reaction between the cationically active species obtained in Step 2-2 and an amine.

Hereinafter, each of the above-described steps will be described. The reactions of the methods for producing an amide according to the present invention are not limited to the reactions exemplified in the following steps.

### <Step 1-2>

Step 1-2 is a step of causing a reaction between a carboxylic acid and a haloformic acid ester to obtain a mixed acid anhydride.

In Step 1-2 of the method for producing an amide according to the embodiment, a carboxylic acid represented by the following General Formula (1) and a haloformic acid ester represented by the following General Formula (I)' are caused to react to obtain a mixed acid anhydride represented by the following General Formula (II). 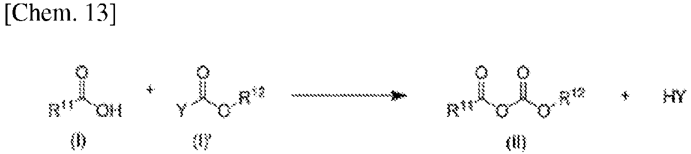 (In the formula, R¹¹ represents a hydrogen atom or a monovalent organic group, R¹² represents a hydrogen atom or a hydrocarbon group, and Y represents a halogen atom.)

Examples of the carboxylic acid include those exemplified in <First Embodiment> described above.

Halogenation of a haloformic acid ester indicates that halogen atoms are bound to carbonyl groups.

A hydrocarbon group of R¹² may be an aliphatic hydrocarbon group or may be an aromatic hydrocarbon group (aryl group). The aliphatic hydrocarbon group may be a saturated aliphatic hydrocarbon group (alkyl group) or may be a unsaturated aliphatic hydrocarbon group, and is preferably an alkyl group.

The aliphatic hydrocarbon group may have 1 to 20 carbon atoms or 1 to 15 carbon atoms.

The alkyl group may be either linear, branched, or cyclic. In the case of the cyclic shape, the alkyl group may be either monocyclic or polycyclic. The alkyl group may have 1 to 20 carbon atoms, 1 to 10 carbon atoms, or 1 to 5 carbon atoms.

Examples of the linear or branched alkyl group include a methyl group, an ethyl group, an n-propyl group, an isopropyl group, an n-butyl group, an isobutyl group, an sec-butyl group, a tert-butyl group, an n-pentyl group, an isopentyl group, a neopentyl group, a tert-pentyl group, a 1 -methylbutyl group, an n-hexyl group, a 2-methylpentyl group, a 3-methylpentyl group, a 2,2-dimethylbutyl group, a 2,3-dimethylbutyl group, an n-heptyl group, a 2-methylhexyl group, a 3-methylhexyl group, a 2,2-dimethylpentyl group, a 2,3-dimethylpentyl group, a 2,4-dimethylpentyl group, a 3,3-dimethylpentyl group, a 3-ethylpentyl group, a 2,2,3-trimethylbutyl group, an n-octyl group, an isooctyl group, a nonyl group, a decyl group, a undecyl group, a dodecyl group, a tridecyl group, a tetradecyl group, a pentadecyl group, a hexadecyl group, a heptadecyl group, an octadecyl group, a nonadecyl group, and an icosyl group.

The halogen atom of Y is an element such as F, Cl, Br, or I belonging to Group 17 in the periodic table, and Cl or Br is preferable.

From the viewpoint of more effectively suppressing side reactions, it is preferable that, in the haloformic acid ester represented by General Formula (I)', the halogen atom of Y be Cl or Br and the hydrocarbon group of R¹² be a branched alkyl group having 1 to 5 carbon atoms, and it is more preferable that the haloformic acid ester be any one or more kinds selected from the group consisting of isopropyl chloroformate, isobutyl chloroformate, ethyl chloroformate, isopropyl bromoformate, isobutyl bromoformate, and ethyl bromoformate.

It is also possible to make the reaction of Step 1-2 easily proceed by causing a reaction between a second base that activates the haloformic acid ester and the haloformic acid ester. Here, an activated haloformic acid ester is also included in the concept of a haloformic acid ester.

The second base reacts with the haloformic acid ester to produce a cationically active species, and is preferably one preferentially reacting with the haloformic acid ester than the mixed acid anhydride which is a product of Step 1-2 and more preferably any one or more kinds selected from the group consisting of tertiary amines, 4-methylmorpholine, pyridine, pyridine derivatives, imidazole, imidazole derivatives, and 1,4-diazabicyclo[2,2, 2]octane. Examples of pyridine derivatives and imidazole derivatives include those exemplified in Step 2-1. Tertiary amines in which at least one of groups binding to N atoms of the amines is a methyl group are preferable. Tertiary amines in which two groups binding to N atoms of the amines are methyl groups are more preferable. If at least one of the groups binding to N atoms of tertiary amines is a methyl group, steric hindrance around N atoms can be reduced and the reaction efficiency with the haloformic acid ester can be improved.

From the above viewpoint, preferred examples of second bases include 4-methylmorpholine, N-methylimidazole, N,N-dimethylbenzylamine, N,N-dimethylbutylamine, N,N-dimethylallylamine, 1,4-diazabicyclo[2,2, 2]octane, N,N-dimethyl-4-aminopyridine (DMAP), and N-methylpiperidine, but the present invention is not limited thereto.

From the viewpoint of yield, N,N-dimethylbenzylamine is preferably used. There is a concern that addition of excessive amount of second base to a reaction system may cause precipitation (formation of salts) due to addition of acids. In this respect, since N,N-dimethylbenzylamine has favorable reactivity, even if the equivalent weight is lowered, its reaction easily proceeds. Therefore, the problem of precipitation is unlikely to occur. For this reason, N,N-dimethylbenzylamine is suitable for a production method in which a continuous flow reactor that is easily affected by precipitation is used.

### <Step 2-2>

Step 2-2 is a step of causing a reaction between the mixed acid anhydride obtained in Step 1-2 and a first base through addition of an acid to obtain a cationically active species.

In Step 2-2 of the method for producing an amide according to the embodiment, a mixed acid anhydride represented by the following General Formula (II) and a first base represented by B are caused to react with each other through addition of an acid represented by A to obtain a cationically active species represented by the following General Formula (IV). In the reaction, a compound represented by the following General Formula (V) is produced as a counter anion of a cationically active species. 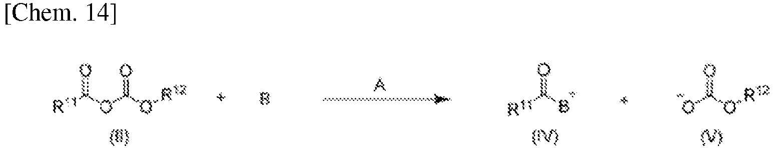 (In Formulae (IV) and (V), R¹¹ and R¹² have the same meanings as R¹¹ and R¹² of the above-described Formula (II).)

Examples of the acid include those exemplified in <First Embodiment> described above.

Examples of the first base include those exemplified in <First Embodiment> described above.

By adding an acid, epimer production of amides can be suppressed, the coupling reaction rate between a carboxylic acid and an amine increases, and an amide can be produced with significantly high efficiency.

### <Step 3-2>

Step 3-2 is a step of producing an amide by causing a reaction between the cationically active species obtained in Step 2-2 and an amine.

In Step 3-2 of the method for producing an amide according to the embodiment, a cationically active species represented by the following General Formula (IV) and an amine represented by the following General Formula (VI) are caused to react to obtain an amide represented by the following General Formula (VII). Although not shown in the following formula, the acid added in the above-described Step 2-2 may be present in the reaction system. 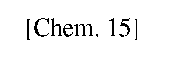 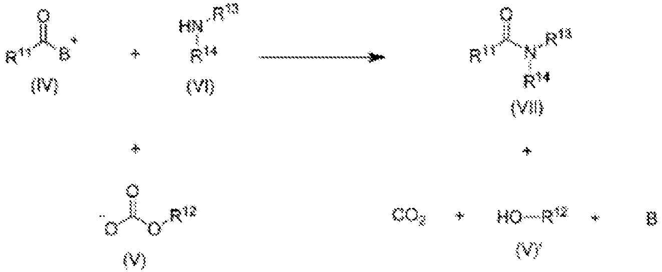 (In Formulae (IV) and (VII), R¹¹ has the same meanings as R¹¹ of the above-described Formula (II). R¹³ and R¹⁴ in Formulae (VI) and (VII) each independently represent a hydrogen atom or a monovalent organic group. R¹² in Formula (V) has the same meaning as R¹² in the above-described Formula (II).)

Examples of the amine include those exemplified in <First Embodiment> described above.

Alkoxide (O⁻-R¹²) and CO₂ may be produced in the above-described Steps 2-2 and 3-2 instead of Formula (V).

### <Reaction conditions>

In the embodiment, the amount of each compound during the reactions of Steps 1 to 3 may be appropriately adjusted depending on a desired reaction in consideration of the types of these compounds.

The molar equivalent ratio (activated carboxylic acid : amine) of an activated carboxylic acid to an amine in a reaction system may be 10:1 to 1/10:1, 5:1 to 1/5:1, and 3:1 to 1/3:1. The activated carboxylic acid is, for example, a compound represented by General Formula (4) or (IV). According to the method for producing an amide of the embodiment, even if a relatively small amount of amine which is close to an equivalent amount is caused to react with an activated carboxylic acid, an amide can be produced with high efficiency.

In the embodiment, the molar equivalent ratio (activated carboxylic acid : acid) of an activated carboxylic acid to an acid in a reaction system may be 10:0.1 to 10:5 or 10:0.5 to 10:4 from the viewpoint of a reaction efficiency.

In the embodiment, the reaction time of each step may be appropriately adjusted depending on other conditions such as a reaction temperature. For example, the reaction time of Step 1 may be 0.05 seconds to 30 minutes, 0.1 seconds to 5 minutes, or 0.5 seconds to 30 seconds. In a case where Steps 2 and 3 are performed at the same time, the reaction time of Steps 2 and 3 may be 1 second to 60 minutes, 5 seconds to 30 minutes, or 1 minute to 10 minutes.

In the embodiment, the temperature (reaction temperature) during the reactions of Steps 1 to 3 may be appropriately adjusted depending on the types of compounds used in Steps 1 to 3. As an example, the reaction temperature is preferably within a range of 0°C to 100°C and more preferably within a range of 20°C to 60°C.

In the present embodiment, the reactions of Steps 1 to 3 may be performed in the coexistence of a solvent. Although the solvent is not particularly limited, one that does not interfere with the reactions between the compounds is preferable and one having high solubility for a raw material to be used in the reactions is preferable. For example, N,N-dimethylformamide (DMF), tetrahydrofuran (THF), 1,4-dioxane, acetonitrile, and dimethoxyethane are exemplified.

In the present embodiment, the reactions of Steps 1 to 3 may further include other compounds that do not correspond to the compounds exemplified above in the reaction system within a range in which the production of amides can be achieved.

In the present embodiment, the reactions of Steps 1 to 3 may be performed separately or simultaneously. Steps 2 and 3 are preferably performed simultaneously from the viewpoint of more effectively suppressing formation of side reaction products.

In the above-described method for producing an amide, the presence and structure of products can be checked through, for example, element analysis or measurement of spectra obtained through NMR, IR, or analysis of mass or the like. In addition, products may be purified as necessary and can be purified through distillation, extraction, recrystallization, column chromatography, or the like as the purification method.

According to the method for producing an amide of the embodiment, amides can be produced with significantly high efficiency. Even the acid anhydride obtained in Step 1 accepts a nucleophilic species (amine) as an active species. In this method, a cationically active species is further formed in Step 2, and the amine is first caused to react with the formed cationically active species. Since the cationically active species formed here has a significantly high activity compared to the acid anhydride, the reaction can proceed at a very high rate and production of by-products can also be dramatically suppressed compared with conventional methods. Furthermore, it is possible to easily allow a reaction to proceed even with an amine having low reactivity which has been difficult to cause a reaction in the conventional method.

According to the method for producing an amide of the embodiment, addition of an acid to a reaction system can probably improve efficiency of production of an intermediate (For example, a cationically active species) derived from a carboxylic acid and can probably suppress epimer production of amides. The mechanism by which these effects occur can be considered, for example, as follows.

One is that it is thought that the addition of an acid supplies protons to a reaction system, whereby racemization of an intermediate derived from a carboxylic acid which is a raw material is suppressed. Racemization can occur when a proton-desorbed anion has a stable structure and a proton is added thereto again. That is, it is thought that racemization is more likely to occur under basic conditions and is suppressed by adding an acid to bring it closer to neutral conditions.

Regarding this, it is thought that the addition of an acid also neutralizes a surplus base, whereby racemization of an intermediate derived from a carboxylic acid which is a raw material is suppressed. It is thought that, in a case where an intermediate (for example, a cationically active species) derived from a carboxylic acid which is a raw material is an intermediate produced through a reaction with a base and a proton-desorbed anion is a stable structure, the racemization in this case is suppressed by inhibiting the reaction with a base due to addition of an acid. This is understood as a case where a side reaction is suppressed in Step 2 and/or Step 3.

Alternatively, it is thought that the efficiency of a coupling reaction between a carboxylic acid and an amine is enhanced due to complex factors due to addition of an acid, whereby a reaction of epimer production of amides is relatively suppressed. This is understood as a case where a side reaction is suppressed in Step 3.

As described above, according to the method for producing an amide of the embodiment, epimer production can be suppressed and amides can be produced with significantly high efficiency.

### «Method for Producing Peptide»

In the method for producing an amide of the embodiment, in a case where the carboxylic acid is an amino acid or an amino acid derivative and the amine is an amino acid or an amino acid derivative, a peptide or a protein can be synthesized. The method for producing a peptide or a protein includes the method for producing an amide.

The amide obtained in the above-described Step 3 can be further repeatedly subjected to Steps 1 to 3 after Steps 1 to 3 using a carboxylic acid of Step 1 to extend polypeptide chains.

That is, the carboxylic acid also includes a polypeptide and an amino acid or an amino acid derivative (carboxylic acid) according to the embodiment also includes an amino acid or an amino acid derivative (carboxylic acid) positioned at the C-terminus as a constitutional unit of a polypeptide. In this manner, the method for producing an amide of the embodiment is suitable as a method for producing a peptide or a protein.

### << Continuous Flow Reactor>>

The method for producing an amide of the embodiment can be carried out with a continuous flow reactor. For example, a continuous flow reactor includes: a flow path for transporting a fluid containing an intermediate or a raw material used for a reaction in the method for producing an amide of the embodiment; and a mixer for mixing the fluid. Regarding the use of the continuous flow reactor, a reaction with an amine may be performed in a continuous flow reactor in at least Step 3, a reaction with a first base and a reaction with an amine may be performed in a continuous flow reactor in Steps 2 and 3, or a reaction with a first base and a reaction with an amine may be performed in a continuous flow reactor in Steps 1 to 3 after obtaining an acid anhydride, for example. The method for producing an amide of the embodiment is not limited to be carried out with a continuous flow reactor. For example, a batch container having a small volume and a high stirring rate may be used. The volume of a mixing portion of the batch container may be 1 to 100 mL or 5 to 50 mL.

Hereinafter, the form of the continuous flow reactor according to the embodiment and the method for producing an amide of the above-described second embodiment in which the form is used will be described with reference to Fig. 1.

Fig. 1 is a schematic diagram illustrating a schematic configuration of a continuous flow reactor 1. The continuous flow reactor 1 includes a tank 11 for accommodating a first liquid, a tank 12 for accommodating a second liquid, and a tank 13 for accommodating a third liquid.

As an example, the first liquid can contain a carboxylic acid, the second liquid can contain a haloformic acid ester, and the third liquid can contain a first base, a liquid, and an amine. As another example, the first liquid can contain a carboxylic acid and a second base that activates a haloformic acid ester, the second liquid can contain a haloformic acid ester, and the third liquid can contain a first base, a liquid, and an amine. As a more specific example, as shown in Fig. 1, the first liquid contains a carboxylic acid, N,N-dimethylbenzylamine (second base), and DIEA, the second liquid contains isopropyl chloroformate, and the third liquid contains N-methylimidazole (first base), a hydrochloric acid (acid), and an amine.

Regarding the continuous flow reactor, mixing of a mixture of at least a first liquid and a second liquid with a third liquid may be performed in the continuous flow reactor, and mixing of a first liquid with a second liquid may be performed in the continuous flow reactor, for example.

The continuous flow reactor 1 includes flow paths f1, f2, f3, f4, and f5 for transporting a fluid. The inner diameters of the flow paths may be, for example, 0.1 to 10 mm or 0.3 to 8 mm. The continuous flow reactor 1 includes mixers 31 and 32 for mixing the fluids. The inner diameters of the flow paths inside the mixers may be, for example, 0.1 to 10 mm or 0.3 to 8 mm. Examples of mixers include a static mixer having no driving portion. The driving portion refers to a portion that is powered and moves.

The above-described inner diameters of the flow paths can be regarded as diameters of portions (portions through which fluids pass) inside the flow paths in the cross sections of the flow paths in directions perpendicular to the longitudinal directions of the flow paths. In a case where the shapes of the portions inside the flow paths are not perfect circles, the above-described inner diameters of the flow paths can be regarded as diameters when the shapes of the above-described portions inside the flow paths are converted into perfect circles based on their areas.

Tanks 11, 12, 13, and 14, the mixers 31 and 32, and the flow paths f1, f2, f3, f4, and f5 are made of, for example, a resin such as plastic or an elastomer, a glass material, a metal, or a ceramic.

The tank 11 is connected to a pump 21, and a first liquid accommodated in the tank 11 moves in the flow path f1 and flows into the mixer 31 due to an operation of the pump 21. The tank 12 is connected to a pump 22, and a second liquid accommodated in the tank 12 moves in the flow path f2 and flows into the mixer 31 due to an operation of the pump 22. The first liquid and the second liquid are mixed by the mixer 31 to become a first mixed liquid mixed using the mixer 31 and sent to the flow path f4.

Dehydration condensation is caused between a carboxylic acid contained in a first liquid and isopropyl chloroformate contained in a second liquid in the process after this mixing to obtain a mixed acid anhydride (Step 1-2 of the method for producing an amide). The first mixed liquid containing the obtained acid anhydride flows into the mixer 32.

On the other hand, the tank 13 is connected to a pump 23, and a liquid accommodated in the tank 13 moves in the flow path f3, flows into the mixer 32 due to an operation of the pump 23, mixed with the first mixed liquid to become a second mixed liquid, and sent to the flow path f5. In the process after this mixing, the mixed acid anhydride obtained in Step 1-2 reacts with N-methylimidazole contained in the third liquid to become a cationically active species (Step 2-2 of the method for producing an amide), and subsequently, the obtained cationically active species reacts with the amine contained in the third liquid to obtain an amide (Step 3-2 of the method for producing an amide). The second mixed liquid containing the produced amide is stored in the tank 14.

According to the continuous flow reactor 1 of the embodiment, the area for heat exchange per volume of a reaction solution can be increased. In addition, the reaction time can be controlled by the flow rate or the length of a flow path. For this reason, the reaction solution can be strictly controlled. As a result, the progress of an undesired side reaction can be minimized and the yield of a target product can be improved.

Although there is an advantage that the cationically active species obtained in Step 2 can be reacted even with an amine having low reactivity due to high activity, control of the reaction is important. In addition, even the acid anhydride obtained in Step 1 has sufficiently high activity, and therefore, control of the reaction is important.

According to the continuous flow reactor 1 of the embodiment, by continuously circulating a liquid through the flow paths, chances of collision of compounds can be increased, the reaction can proceed more efficiently, and suppression of side reactions is facilitated. For example, the acid anhydride produced in Step 1 can be caused to immediately react with N-methylimidazole (first base), and therefore, the time during which the acid anhydride is in an activated state can be shortened and the probability that a side reaction such as isomerization can be reduced.

Although the form in which liquids are mixed with each other with mixers has been exemplified in the continuous flow reactor according to the present embodiment, since mixing of liquids can be achieved simply through communication between flow paths, the continuous flow reactor of the embodiment may not necessarily include mixers.

In the continuous flow reactor exemplified above, the method for producing an amide of the second embodiment has been described as an example, but the first embodiment of the method for producing an amide can also be similarly carried out.

As an example, a first liquid can contain a first carboxylic acid and a second carboxylic acid, a second liquid can contain phosgene or a phosgene equivalent that decomposes in a reaction system and produces phosgene, and a third liquid can contain a first base, an acid, and an amine. As a more specific example, a first liquid can contain a carboxylic acid and DIEA, a second liquid can contain triphosgene (phosgene equivalent), and a third liquid can contain 4-morpholinopyridine (first base), hydrochloric acid (acid), and an amine.

As shown here, the method for producing an amide of the embodiment can be carried out through a liquid phase method. For example, the current mainstream method for producing a peptide (amide) is a solid phase method, and a peptide is synthesized on a solid phase. On the other hand, the liquid phase method is suitable for large-scale synthesis, and reactivity is also favorable due to a high degree of freedom of molecules. The liquid phase method is also effective for a reaction with an amine having low reactivity.

Five kinds of compounds to be reacted have been divided and accommodated in three tanks in the continuous flow reactor according to the present embodiment. However, the five compounds may be accommodated in five separate tanks and sequentially mixed with each other, for example.

However, as shown as the third liquid of the above-described embodiment, it is preferable that N-methylimidazole (first base) and an amine be present in the same liquid in advance. That is, Step 2 and Step 3 may be performed at the same time. Accordingly, the cationically active species having high reactivity produced in Step 2 is easily immediately caused to react with a target amine, whereby the time during which the cationically active species is in an activated state can be shortened and production of undesired side reaction products can be effectively suppressed.

In addition, as shown as the third liquid of the above-described embodiment, it is preferable that N-methylimidazole (first base) and an acid be present in the same liquid in advance. Accordingly, production of undesired epimers can be effectively suppressed.

The embodiments of this invention have described in detail above with reference to the chemical formulae and drawings. However, the configurations, the combinations thereof, and the like in the above-described embodiments are merely examples, and addition, omission, replacement, and other modifications of the configurations can be made within the scope not departing from the gist of the present invention. In addition, the present invention is not limited by each embodiment, but is limited only by the scope of the claims.

### [Examples]

Examples will be shown below to describe the present invention in more detail, but the present invention is not limited to the following examples.

### <Example 1> Addition of HCl in Mixed Acid Anhydride Method

### [Raw Material]

Fmoc-2-Phenylglycine-OH (commercially available product) which was phenylglycine having an amino group protected by a Fmoc group was used as an amino acid used as a carboxylic acid. H-MePhe-OMe (commercially available product) which was amino group-methylated phenylalanine having a carboxyl group protected by a methyl group was used as an amino acid used as an amine.

### [Flow Synthesis of Acid Amide]

A coupling reaction between an amino acid used as a carboxylic acid and an amino acid used as an amine was performed. A continuous flow reactor consisting of a PTFE tube (inner diameter of 0.8 mm, outer diameter of 1.59 mm), a SUS tube (inner diameter of 0.8 mm, outer diameter of 1.58 mm), a SUS joint, and a T-shaped mixer was used for the coupling reaction. Solutions before the reaction were adjusted by being divided into three. A first solution was obtained by dissolving Fmoc-2-Phenylglycine-OH used as a carboxylic acid, DIEA, and N,N-dimethylbenzylamine used as a second base in 1,4-dioxane. A second solution was obtained by dissolving isopropyl chloroformate in 1,4-dioxane. A third solution was obtained by dissolving H-MePhe-OMe used as an amine, N-methylimidazole used as a first base, and HCl in a mixed solvent of 1,4-dioxane and MeCN. The ratio of each molar concentration in the continuous flow reactor was 1.1 for Fmoc-2-Phenylglycine-OH (carboxylic acid), 1.1 for DIEA, 0.055 for N,N-dimethylbenzylamine, 1.05 for isopropyl chloroformate, 0.1 for N-methylimidazole, and 0.155 for HCl with respect to 1.0 of H-MePhe-OMe (amine).

In order to perform the coupling in the continuous flow reactor, the first solution was mixed with the second solution with a T-shaped mixer to cause a reaction for 5 seconds in the continuous flow reactor, and a mixed acid anhydride was obtained. Thereafter, the reaction solution containing the mixed acid anhydride was immediately mixed with the third solution with a new T-shaped mixer to cause a reaction for 120 seconds in the continuous flow reactor. All of these reactions were carried out at 60°C, and the time for performing heat exchange before the solutions before the reactions reached the mixers was set to 10 seconds. Various solutions flowed out using syringe pumps. The flow rate of each pump was 2.0 mL/min for the first solution, 1.2 mL/min for the second solution, and 2.0 mL/min for the third solution.

The reaction of Step 1 of the method for producing an amide of Example 1 is shown below. 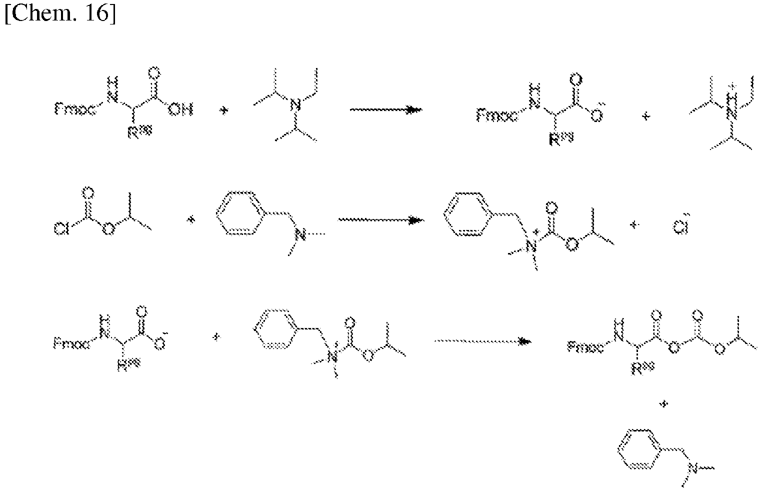 [In the formula, R^{pg} represents a phenylglycine side chain.]

The reactions of Steps 2 and 3 of the method for producing an amide of Example 1 are shown below. 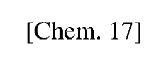 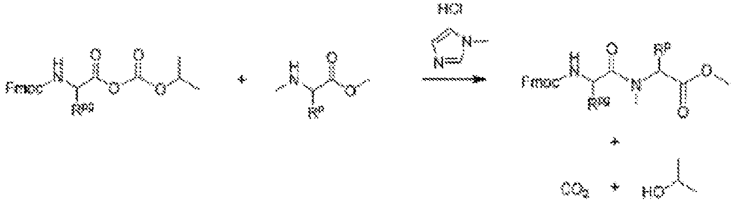 [In the formula, R^{pg} represents a phenylglycine side chain and R^{p} represents a phenylalanine side chain.]

### [Analysis Method]

Target products were isolated through silica gel column chromatography and identified through NMR analysis.

The yield of the target products were calculated from the weight of the target products which had been isolated and purified. That is, the molar equivalent ratio of amines was set to 1.0, and the proportion at which the amines were coupled was calculated from the weight of isolated dipeptides.

Isomers of the isolated and purified amides were separated by HPLC, and the epimer production rate was calculated from area ratios of UV absorption intensity of the amides of the target products to the epimers. The target products were detected at a retention time of 35.576 minutes (area ratio of 73.58) and the epimers were detected at a retention time of 39.658 minutes (area ratio of 26.42).

### [Results]

The NMR data of the obtained dipeptides is shown below. ¹H NMR (500 MHz, CDCl₃): δ 7.78-7.70 (m, 2H), 7.59-7.51 (m, 2H), 7.46-6.84 (m, 14H), 6.28 (d, J = 7.5 Hz, 1H), 5.48 (d, J = 7.5 Hz, 1H), 5.17-5.11 (m, 1H), 4.36-4.23 (m, 2H), 4.20-4.14 (m, 1H), 3.68 (s, 3H), 3.42-3.35 (m, 1H), 3.05-2.95 (m, 1H), 2.70 (s, 3H)

As a result of analyzing products after the reaction, the dipeptides which were the target products had a coupling yield of 86%, and of these the epimer production rate was about 23%.

According to the method of Example 1, the molar concentration ratio of carboxylic acids to amines is 1:1.1, and a high coupling yield of 86% could achieved despite a short reaction time of 120 seconds. In addition, despite the fact that phenylglycine which was very likely to cause epimer production was used as a carboxylic acid, the production rate of epimers can be greatly reduced compared to Comparative Example 1 to be described below.

### <Comparative Example 1> Mixed Acid Anhydride Method

### [Raw Material]

Fmoc-2-Phenylglycine-OH (commercially available product) which was phenylglycine having an amino group protected by a Fmoc group was used as an amino acid used as a carboxylic acid. H-MePhe-OMe (commercially available product) which was amino group-methylated phenylalanine having a carboxyl group protected by a methyl group was used as an amino acid used as an amine.

### [Flow Synthesis of Acid Amide]

A coupling reaction between an amino acid used as a carboxylic acid and an amino acid used as an amine was performed. A continuous flow reactor consisting of a PTFE tube (inner diameter of 0.8 mm, outer diameter of 1.59 mm), a SUS tube (inner diameter of 0.8 mm, outer diameter of 1.58 mm), a SUS joint, and a T-shaped mixer was used for the coupling reaction. Solutions before the reaction were prepared by being divided into three. A first solution was obtained by dissolving Fmoc-2-Phenylglycine-OH used as a carboxylic acid, N-methylmorpholine, and DIEA in 1,4-dioxane. A second solution was obtained by dissolving isopropyl chloroformate in 1,4-dioxane. A third solution was obtained by dissolving H-MePhe-OMe used as an amine, and 4-morpholinopyridine in 1,4-dioxane. The ratio of each molar concentration in the continuous flow reactor was 1.3 for Fmoc-2-Phenylglycine-OH (carboxylic acid), 1.3 for DIEA, 1.3 for N-methylmorpholine, 1.25 for isopropyl chloroformate, and 0.01 for 4-morpholinopyridine with respect to 1.0 of H-MePhe-OMe (amine).

In order to perform the coupling in the continuous flow reactor, the first solution was mixed with the second solution with a T-shaped mixer to cause a reaction for 5 seconds in the continuous flow reactor, and a mixed acid anhydride was obtained. Thereafter, the reaction solution containing the mixed acid anhydride was immediately mixed with the third solution with a new T-shaped mixer to cause a reaction for 150 seconds in the continuous flow reactor. All of these reactions were carried out at 60°C, and the time for performing heat exchange before the solutions before the reactions reached the mixers was set to 10 seconds. Various solutions flowed out using syringe pumps. The flow rate of each pump was 1.2 mL/min for the first solution, 1.2 mL/min for the second solution, and 2.0 mL/min for the third solution.

### [Analysis Method]

The analysis method was performed through the same method as that in Example 1.

### [Results]

As a result of analyzing products after the reaction, because the dipeptides which were the target products had a coupling yield of 93%, of these the epimer production rate was about 41%, and nearly half of them were epimers, it became clear that the epimer production had not been mostly suppressed.

As a result, in Comparative Example 1, it was difficult to suppress epimer production in the case where phenylglycine was used as a carboxylic acid.

### [Reference Signs List]

1 Continuous flow reactor
11, 12, 13, 14 Tank
21, 22, 23 Pump
31, 32 Mixer
f1, f2, f3, f4, f5 Flow path

## Claims

1. A method for producing an amide, the method comprising:
subjecting carboxylic acids to dehydration condensation or causing a reaction between a carboxylic acid and a haloformic acid ester; and
subsequently causing a reaction with a first base and a reaction with an amine to obtain an amide,
wherein the reaction with a first base and/or the reaction with an amine is performed by adding an acid thereto.

2. The method for producing an amide, the method comprising:
mixing a product obtained by reacting a mixture obtained by mixing a first carboxylic acid with a second carboxylic acid or a product obtained by reacting a mixture obtained by mixing a carboxylic acid and a haloformic acid ester, with a first base, an acid, and an amine.

3. The method for producing an amide according to claim 1 or 2,
wherein phosgene or a phosgene equivalent that decomposes in a reaction system and produces phosgene is caused to react, and the carboxylic acids are subjected to dehydration condensation.

4. The method for producing an amide according to any one of claims 1 to 3,
wherein the same kinds of the carboxylic acids are subjected to dehydration condensation.

5. The method for producing an amide according to claim 1 or 2,
wherein the haloformic acid ester is any one or more kinds selected from the group consisting of isopropyl chloroformate, isobutyl chloroformate, ethyl chloroformate, isopropyl bromoformate, isobutyl bromoformate, and ethyl bromoformate.

6. The method for producing an amide according to claim 1, 2, or 5, the method further comprising:
causing a reaction between the haloformic acid ester and a second base that activates the haloformic acid ester.

7. The method for producing an amide according to claim 6,
wherein the second base is any one or more kinds selected from the group consisting of tertiary amines, 4-methylmorpholine, pyridine, pyridine derivatives, imidazole, imidazole derivatives, and 1,4-diazabicyclo[2,2, 2]octane.

8. The method for producing an amide according to any one of claims 1 to 7,
the acid is any one or more kinds selected from the group consisting of a hydrogen halide, perchloric acid, trifluoroacetic acid, trifluoromethanesulfonic acid, fluorosulfonic acid, and paratoluenesulfonic acid.

9. The method for producing an amide according to any one of claims 1 to 8,
wherein the carboxylic acids are amino acids or amino acid derivatives.

10. The method for producing an amide according to any one of claims 1 to 9,
wherein the carboxylic acids are phenylglycine, cysteine, serine, aspartic acid, histidine, or derivatives thereof.

11. The method for producing an amide according to any one of claims 1 to 10,
wherein the first base is any one or more kinds selected from the group consisting of pyridine, pyridine derivatives, imidazole, imidazole derivatives, and 1,4-diazabicyclo[2, 2, 2]octane.

12. The method for producing an amide according to according to any one of claims 1 to 11,
wherein the first base is any one or more kinds selected from the group consisting of 4-morpholinopyridine, N,N-dimethyl-4-aminopyridine, 4-pyrrolidinopyridine, pyridine, 4-methoxypyridine, imidazole, N-methylimidazole, and 1,4-diazabicyclo[2, 2, 2]octane.

13. The method for producing an amide according to any one of claims 1 to 12, wherein the amine is an amino acid or an amino acid derivative.

14. The method for producing an amide according to any one of claims 1 to 13,
wherein a nucleophilicity of the amine is lower than that of 18 kinds of amino acids excluding valine and isoleucine from 20 kinds of amino acids which constitute proteins and are encoded by genetic information.

15. The method for producing an amide according to claim 13 or 14,
wherein the amine is valine, isoleucine, an N-alkylated amino acid, or derivatives thereof.

16. The method for producing an amide according to any one of claims 1 to 15,
wherein the reaction with the amine is performed in a continuous flow reactor.

17. The method for producing an amide according to claim 16,
wherein the reaction between the carboxylic acids or the reaction between the carboxylic acid and the haloformic acid ester, and the reaction with the first base are further performed in the continuous flow reactor.
